# EUROPÄISCHE PATENTSCHRIFT

(11) **EP 3 967 351 B1**
(45) Veröffentlichungstag und Bekanntmachung des Hinweises auf die Patenterteilung: **13.12.2023**
(21) Anmeldenummer: 21000217.6
(22) Anmeldetag: 09.08.2021
(51) Int. Cl.: A61M 16/06, A61M 16/04

(54) **MASKENWULST MIT SCHLAUCHDURCHFÜHRUNG**
MASK BEAD WITH TUBE FEEDTHROUGH
JUPE DE MASQUE POURVU DE PASSAGE DE TUYAU

(30) Priorität: 11.09.2020 DE 102020123713
(43) Veröffentlichungstag der Anmeldung: 16.03.2022
(73) Patentinhaber: Löwenstein Medical Technology S.A., 2557 Luxembourg (LU)
(72) Erfinder: Eifler, Martin, 25348 Glückstadt (DE)
(74) Vertreter: Marx, Thomas

(56) Entgegenhaltungen:
- EP-A1- 3 259 005
- WO-A1-2015/130179
- WO-A1-2018/097470
- US-A- 4 328 797
- US-A1- 2008 230 072
- US-A1- 2011 186 051
- US-B2- 10 004 864

## Beschreibung

Die Erfindung betrifft einen Maskenwulst für ein Patienteninterface zur optionalen Anwendung mit einem Schlauch zwischen Maskenwulst und Gesicht eines Patienten.

In manchen Fällen benötigen Patienten, welche beatmet werden oder bei der Atmung unterstützt werden müssen, zusätzlich noch eine Magensonde um eine Versorgung mit Nährstoffen, Flüssigkeit oder Medikamenten zu gewährleisten. Die Magensonde wird dabei in der Regel mittels Schlauch durch den Mund oder die Nase appliziert. Bei konventionellen Beatmungsmasken entsteht bei einer Führung eines Schlauches zwischen Maske und Gesicht des Patienten eine hohe Leckage. In der Regel kann diese Leckage durch ein stärkeres Festschnallen der Maske auf dem Gesicht des Patienten nur geringfügig verringert werden, fördert aber zugleich die Entstehung von Druckstellen und kann zu einem unbequemen Tragen der Maske führen.

Um das Tragen einer Beatmungsmaske bei gleichzeitiger Verwendung einer Magensonde zu verbessern gibt es im Stand der Technik verschiedene Ansätze. Ein Ansatz verfolgt dabei Schlauchdurchführungen im Maskenkörper. Diese Lösung erreicht zwar eine gute Abdichtung der Maske, allerding ist es nicht möglich die Maske oder den Schlauch zu wechseln oder zu entfernen ohne das jeweils andere ebenfalls für eine längere Zeit zu entfernen. Eine andere Lösung stellt ein separates Kissen für den Schlauch dar, welches zwischen Maske und Gesicht des Patienten gelegt wird. Ein solches Kissen kann schnell als Fremdkörper wahrgenommen werden und verhindert dazu einen optimalen, bequemen Sitz der Maske. Ausschnitte mit Lamellen innerhalb des Ausschnitts in den Seitenbereichen des Maskenkissens ermöglichen zwar eine verbesserte Abdichtung bei Verwendung eines Schlauches, nach Entfernen des Schlauches stellen diese Ausschnitte aber eine zusätzliche Undichtigkeit dar. WO 2015/130 179 A1 offenbart einen Maskenwulst für ein Patienteninterface mit einer Lippe, die einen Abschnitt zur Führung eines Schlauches eines Anwenders aufweist.

Aufgabe der vorliegenden Erfindung ist daher die Bereitstellung eines Maskenwulstes für ein Patienteninterface welches ein einfaches Wechseln oder Entfernen des Patienteninterfaces oder des Schlauches ermöglicht und dabei optimal abdichtend und bequem zu tragen ist. Die Aufgabe wird durch den erfinderischen Maskenwulst nach Anspruch 1 gelöst.

Die Erfindung betrifft einen Maskenwulst für ein Patienteninterface, mit zumindest einer Lippe, wobei die zumindest eine Lippe sich ausgehend von einem Übergangsbereich zu einer zentralen Öffnung hin erstreckt, wobei die mindestens eine Lippe die zentrale Öffnung umrandet, wobei mindestens eine Lippe zumindest einen Abschnitt aufweist, welcher zur Führung eines Schlauches zwischen Maskenwulst und der Gesichtshaut eines Anwenders dient. Erfindungsgemäß weist der Maskenwulst zumindest zwei Lippen auf, wobei mindestens eine Lippe als Dichtlippe und mindestens eine Lippe als Stützlippe ausgebildet ist wobei die Stützlippe den mindestens einen Abschnitt aufweist.

In manchen Ausführungsformen des Maskenwulstes ist der Übergangsbereich ein Maskenkörperanschluss.

In manchen Ausführungsformen des Maskenwulstes ist das Material der Dichtlippe im Bereich des Abschnittes verformbar und passt sich mindestens teilweise dem Schlauch an.

In manchen Ausführungsformen des Maskenwulstes ist der Abschnitt ein Ausschnitt, welcher die Oberfläche der Stützlippe mindestens stellenweise unterbricht.

In manchen Ausführungsformen des Maskenwulstes liegt die Dichtlippe, ohne Verwendung eines Schlauches zwischen Maskenwulst und Gesicht des Patienten, dichtend auf dem Gesicht des Patienten auf.

In manchen Ausführungsformen des Maskenwulstes ist die Lippe kombiniert als Dichtlippe und Stützlippe ausgebildet.

In manchen Ausführungsformen des Maskenwulstes weisen die Seiten des Ausschnittes ohne mechanische Belastung einen Abstand einer Breite (B) auf.

In manchen Ausführungsformen des Maskenwulstes ist die Dichtlippe dazu eingerichtet, dichtend auf dem Gesicht eines Patienten aufzuliegen.

In manchen Ausführungsformen des Maskenwulstes sind mindestens zwei Lippen benachbart angeordnet und verlaufen zumindest streckenweise parallel zueinander.

In manchen Ausführungsformen des Maskenwulstes ist die Dichtlippe außen angeordnet und weist eine weitestgehend geringere Materialstärke als die Stützlippe auf, wobei die Stützlippe innen angeordnet ist.

In manchen Ausführungsformen des Maskenwulstes liegt die Stützlippe außen und weist eine weitestgehend höhere Materialstärke als die Dichtlippe auf.

In manchen Ausführungsformen des Maskenwulstes weist die Dichtlippe einen als Dünnstelle ausgebildeten Abschnitt auf, welcher eine geringere Materialstärke als der Bereich der Dichtlippe, der den Abschnitt umgibt, aufweist.

In manchen Ausführungsformen des Maskenwulstes weist die Stützlippe einen als Ausschnitt ausgebildeten Abschnitt auf.

In manchen Ausführungsformen des Maskenwulstes weist der Abschnitt zumindest ein Ende in Richtung der zentralen Öffnung sowie ein Ende in Richtung zum Übergangsbereich auf.

In manchen Ausführungsformen des Maskenwulstes ist das Ende des Abschnittes abgerundet. In manchen Ausführungsformen des Maskenwulstes ist das Ende des Abschnittes halbkreisförmig.

In manchen Ausführungsformen des Maskenwulstes ist der Ausschnitt zur zentralen Öffnung hin offen.

In manchen Ausführungsformen des Maskenwulstes die Endpunkte des zur Öffnung gerichteten Endes des Ausschnittes voneinander beabstandet sind.

In manchen Ausführungsformen des Maskenwulstes berühren sich die Seiten des Ausschnitts im Bereich des Endes nicht.

In manchen Ausführungsformen des Maskenwulstes ist der Abschnitt ausgebildet, über einem Schlauch zu liegen und sich zumindest abschnittsweise der Schlauchform anzupassen.

In manchen Ausführungsformen des Maskenwulstes weist die Stützlippe einen Nasenrückenbereich, zwei Seitenbereich sowie einen Basisbereich auf, wobei sich die Stützlippe von den Seitenbereichen in Richtung zentrale Öffnung erstreckt und im Bereich des Nasenrückenbereichs und des Basisbereichs nicht miteinander verbunden ist.

In manchen Ausführungsformen des Maskenwulstes erstreckt sich die Stützlippe von den Seitenbereichen und vom Nasenrückenbereich in Richtung zentrale Öffnung und ist im Bereich des Basisbereichs nicht miteinander verbunden.

In manchen Ausführungsformen des Maskenwulstes erstreckt sich die Stützlippe von den Seitenbereichen und dem Basisbereich in Richtung zentrale Öffnung und ist im Bereich des Nasenrückenbereichs nicht miteinander verbunden.

In manchen Ausführungsformen des Maskenwulstes weist die Lippe mindestens zwei Abschnitte auf.

In manchen Ausführungsformen des Maskenwulstes weist die Stützlippe mindestens zwei Abschnitte auf, wobei je mindestens einer der mindestens zwei Abschnitte in jeweils einem der Bereiche der Stützlippe, welche sich von den Seitenbereichen in Richtung der zentralen Öffnung erstreckt, mindestens teilweise angeordnet ist.

In manchen Ausführungsformen des Maskenwulstes weist die Stützlippe mindestens zwei Abschnitte auf, welche sich, bezogen auf eine Ebene (S) durch den Nasenrückenbereich und den Basisbereich, gegenüberliegen.

In manchen Ausführungsformen des Maskenwulstes weisen die mindestens zwei Abschnitte die gleichen Abmessungen und Geometrien auf.

Es ist darauf hinzuweisen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des erfindungsgemäßen Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Unter einem Beatmungsgerät ist jedwedes Gerät zu verstehen, welches einen Anwender oder Patienten bei der natürlichen Atmung unterstützt, die Beatmung des Anwenders bzw. Patienten übernimmt und/oder zur Atemtherapie dient und/oder anderweitig die Atmung des Anwenders bzw. Patienten beeinflusst. Darunter fallen zum Beispiel, aber nicht ausschließend, CPAPsowie BiPAP-Geräte, Narkose- bzw. Anästhesiegeräte, Atemtherapiegeräte, (klinische, außerklinische oder Notfall-) Beatmungsgeräte, Highflow-Therapiegeräte und Hustenmaschinen. Beatmungsgeräte können auch als Diagnosegeräte für die Beatmung verstanden werden. Diagnosegeräte können dabei allgemein zur Erfassung von medizinischen Parametern eines Patienten dienen. Darunter fallen auch Geräte, welche medizinische Parameter von Patienten in Kombination mit oder ausschließlich die Atmung betreffend, erfassen und optional verarbeiten können.

Als Patienteninterface kann, soweit nicht ausdrücklich anders beschrieben, jeglicher Teil oder verbundene Peripheriegeräte des Beatmungsgerätes verstanden werden, welche zur Interaktion, insbesondere zu Therapie- oder Diagnosezwecken, mit einem Patienten gedacht ist. Insbesondere kann ein Patienteninterface als Maske eines Beatmungsgerätes bzw. eine mit dem Beatmungsgerät verbundene Maske verstanden werden. Diese Maske kann eine Full-Face-Maske, also Nase und Mund umschließende, eine Totalface-Maske (zumindest Nase, Mund und Augen umschließend), ein Beatmungshelm, oder eine Nasenmaske, also eine nur die Nase umschließende Maske, sein. Auch Trachealtuben und sogenannte Nasenbrillen können als Maske eingesetzt werden.

Im Folgenden wird die Erfindung anhand der Figuren 1 bis 17 über beispielhafte Ausführungsformen näher erläutert.
- Fig. 1: Maskenwulst in einer perspektivischen Vorderansicht
- Fig. 2: Maskenwulst mit zwei Lippenlamellen in perspektivischer Hinteransicht
- Fig. 3: Detailansicht des Ausschnittes aus Fig. 2
- Fig. 4: Maskenwulst aus Fig. 2 in Rückansicht
- Fig. 5: Schnitt A-A durch Maskenwulst aus Fig. 4
- Fig. 6: Detail X aus Fig. 6
- Fig. 7: Maskenwulst mit zwei Lippenlamellen in perspektivischer Hinteransicht
- Fig. 8: Detailansicht des Abschnittes 16 aus Fig. 7
- Fig. 9: Maskenwulst aus Fig. 7 in Rückansicht
- Fig. 10: Schnitt A-A durch Maskenwulst aus Fig. 9
- Fig. 11: Detail X aus Fig. 10
- Fig. 12: Maskenwulst mit zwei Lippenlamellen in perspektivischer Hinteransicht
- Fig. 13: Detailansicht des Ausschnittes aus Fig. 12
- Fig. 14: Maskenwulst aus Fig. 12 in Rückansicht
- Fig. 15: Schnitt A-A durch Maskenwulst aus Fig. 14
- Fig. 16: Detail X aus Fig. 15
- Fig. 17: Maskenwulst mit Ausschnitt in Vorderansicht

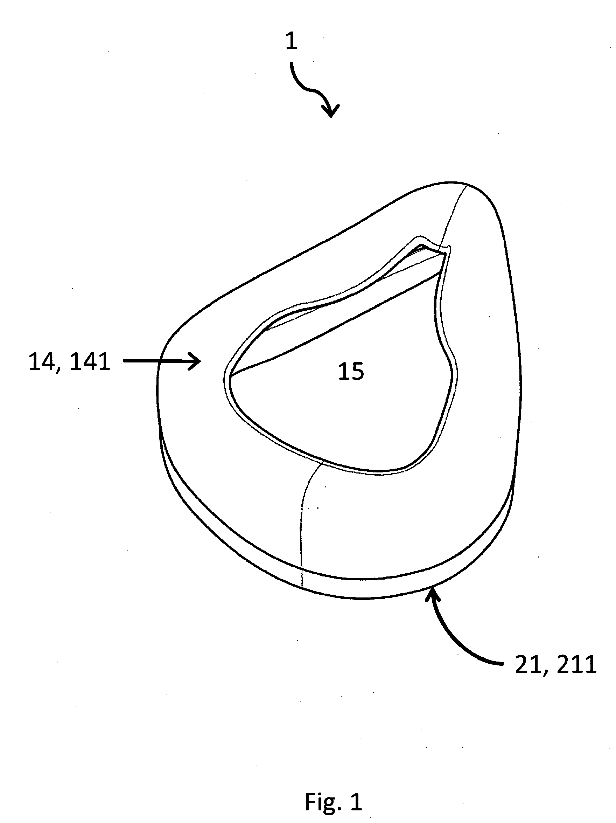

Figur 1 zeigt eine beispielhafte Ausführungsform der Maskenwulst 1 in perspektivischer Vorderansicht. Die Lippe 14 erstreckt sich bei dem Maskenwulst von dem Übergangsbereich 21, welcher hier beispielhaft als Maskenkörperanschluss 211 ausgeführt ist, in Richtung einer zentralen Öffnung 15, welche von der Lippe 14 vollständig umrandet wird. Die Oberfläche 141 der Lippe 14 ist als Kontaktfläche zur Gesichtshaut 4 eines Anwenders ausgebildet und schließt mit dieser, insbesondere ohne Verwendung eines Schlauches 3 zwischen Maskenwulst 1 und Gesichtshaut 4, dichtend ab. Beispielhaft ist die in Fig. 1 gezeigte Ausführungsform ausgebildet um mit einem Maskenkörper des zum Beispiel mehrteilig ausgeführten Patienteninterface 2 verbunden zu werden und verfügt daher über einen als Maskenkörperanschluss 211 ausgebildeten Übergangsbereich 21. In manchen Ausführungsformen ist das Patienteninterface 2 auch einteilig ausgeführt, Maskenwulst 1 und Maskenkörper sind also ein Bauteil, beispielsweise aus einem Material hergestellt. Im Fall eines einteiligen Patienteninterface 2 ist der Übergangsbereich 21 ein beispielsweise fließender Übergang zwischen Maskenwulst 2 und Maskenkörper. Ein einteiliges Patienteninterface 2 kann beispielsweise im 2K-Verfahren und/oder durch anspritzen hergestellt werden. In manchen Ausführungsformen ist der Übergangsbereich 21 mit einer größeren Materialstärke und höherer Steifigkeit als die Lippe 14 ausgeführt. Die Lippe 14 kann beispielsweise als kombinierte Stützlippe 143 und Dichtlippe 142 ausgeführt sein. Die beiden Funktionen des Stützens (Stützlippe 143) und Abdichtens (Dichtlippe 142) kann auch auf zwei oder mehrere Lippen 14 aufgeteilt werden.

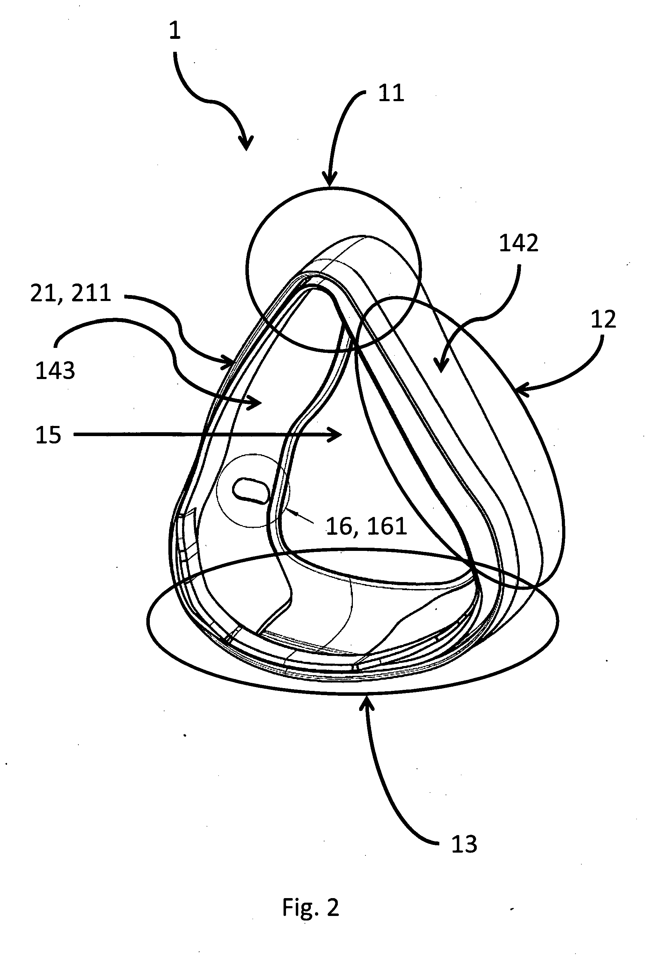

Fig. 2 zeigt eine beispielhafte Ausführungsform der Maskenwulst mit zwei Lippen 14. Dabei ist die äußere Lippe 14 als Dichtlippe 142 ausgeführt. Die innere der beiden Lippen ist als Stützlippe 143 ausgebildet und weist zudem einen Abschnitt 16 auf, welcher zur Führung eines Schlauchs 3 eingerichtet ist. Die Dichtlippe 142 erstreckt sich von dem als Maskenkörperanschluss 211 ausgeführten Übergangsbereich 21 in Richtung der zentralen Öffnung 15 und umrandet diese vollständig. Im Wesentlichen erstreckt sich auch die Stützlippe 143 dem Übergangsbereich 21 zur zentralen Öffnung 15 und umrandet diese zumindest teilweise.

Die Stützlippe 143 weist beispielsweise eine höhere Materialstärke auf als die Dichtlippe 142. Auch ist die Festigkeit bzw. Steifigkeit der Stützlippe 143 höher - etwa als Resultat der höheren Materialstärke - um den Maskenwulst 1 beziehungsweise das Patienteninterface 2 gegenüber dem Gesicht des Anwenders abzustützen. Die Dichtlippe 142 weist beispielsweise im Wesentlichen eine geringe Materialstärke als die Stützlippe 143 auf und kann sich flexibel dem Gesicht des Anwenders anpassen. Die Dichtlippe 142 dient primär dem Abdichten des Maskenwulsts 1 an der Gesichtshaut 4 des Anwenders. In manchen Ausführungsformen kann die Materialstärke der Dichtlippe 142 und/oder der Stützlippe 143 auch graduell vom Übergangsbereich 21 in Richtung der zentralen Öffnung abnehmen und/oder zunehmen. Dadurch kann es vorkommen, dass die Dichtlippe 142 stellenweise eine höhere Materialstärke aufweist als die Stützlippe 143. Ein Bereich der Dichtlippe 142 mit höherer Materialstärke (beispielsweise nah zum Übergangsbereich 21) kann demnach also unter Umständen eine höhere Materialstärke aufweisen als ein Bereich der Stützlippe 143 mit geringer Materialstärke (beispielsweise nah zur zentralen Öffnung 15). Insbesondere bei Ausführungsformen des Maskenwulstes 1 mit nur einer Lippe 14, welche als kombinierte Dichtlippe 142 und Stützlippe 143 ausgebildet ist, kann die Lippe 14 stellenweise in der Materialstärke variieren. Insbesondere im Bereich der Auflagefläche der Dichtlippe 142 auf der Gesichtshaut 4 des Anwenders kann eine besonders geringe Materialstärke der Dichtlippe 142, welche beispielsweise eine einfache Anpassung an die Unebenheiten des Gesichts eines Anwenders ermöglicht.

In Fig. 2 sind weiter der Nasenrückenbereich 11, ein Seitenbereich 12 sowie der Basisbereich 13 angedeutet. Der Nasenrückenbereich 11 ist dabei jener Bereich, welche auf dem Nasenrücken des Anwenders aufliegt. Der Seitenbereich 12 ist sowohl links als auch rechts von Basisbereich 13 und Nasenbereich 11 zu finden, die Markierung des zweiten Seitenbereichs 12 wurde aus Gründen der Übersicht nicht eingezeichnet. Die Seitenbereiche 12 der Stützlippe 143 sind im Fig. 2 dargestellten Bereich des Basisbereiches 13 nicht oder zumindest bereichsweise nicht verbunden. Die Stützlippe umrandet die zentrale Öffnung 15 also, im Gegensatz zu der Dichtlippe 142, nicht vollständig. An der Unterbrechung im Basisbereich 13 der Stützlippe 143 ist gut der Übergang der Lippe 14 zu dem Übergangsbereich 21 zu erkennen. Insbesondere bei einem Patienteninterface 2, welches aus einem Teil gefertigt ist, kann ist es möglich, dass an dieser Stelle ein fließender Übergang ausgebildet ist, dass zum Beispiel die Materialstärke von Lippe 14 zu Übergangsbereich 21 graduell zunimmt. In manchen Ausführungsformen ist es denkbar, dass die Dichtlippe 142 und die Stützlippe 143 zumindest teilweise als eine Lippe 14 vom Übergangsbereich 21 in Richtung zentrale Öffnung 15 verlaufen und sich zwischen Übergangsbereich 21 und zentraler Öffnung 15 in separate Dichtlippe 142 und Stützlippe 143 aufteilen.

In manchen Ausführungsformen sind die Seitenbereiche 12 der Stützlippe 143 auch im Nasenrückenbereich 11 nicht verbunden, sodass die Stützlippe 143 lediglich in den Seitenbereichen 12 ausgebildet ist und höchstens teilweise in dem Basisbereich 13 und/oder dem Nasenrückenbereich 11 ausgebildet ist. In manchen Ausführungsformen sind die Seitenbereiche 12 der Stützlippe 143 auch nur in dem Nasenrückenbereich 11 nicht verbunden. In manchen Ausführungsformen sind die Seitenbereiche 12 der Stützlippe 143 im Nasenrückenbereich 11 und/oder Basisbereich 13 auch zumindest teilweise verbunden. Beispielsweise können zur zentralen Öffnung 15 hin die Seitenbereiche 12 nicht verbunden sein, während die Seitenbereiche 12 der Stützlippe 143 zum Übergangsbereich 21 hin miteinander verbunden sind. Auch ist eine geringere Materialstärke der Stützlippe 143 im Nasenrückenbereich 11 und/oder Basisbereich 13 denkbar.

In einigen beispielhaften Ausführungsformen weist die Dichtlippe 142 im Nasenrückenbereich 11 eine geringere Materialstärke auf als in den anderen Bereichen.

Teilweise ist auch die Materialstärke im Basisbereich 13 der Dichtlippe 142 geringer. In manchen Fällen weisen der Basisbereich 13 und der Nasenrückenbereich 11 die gleiche Materialstärke auf, welche beispielsweise geringer ist als in den Seitenbereichen 12 der Dichtlippe 142. In einigen Ausführungsformen nimmt dazu die Materialstärke der Dichtlippe 142 graduell von den Seitenbereichen 12 zu dem Basisbereich 13 und/oder Nasenrückenbereich 11 ab. In manchen Ausführungsformen können die Dichtlippe 142 und/oder die Stützlippe 143, auch in kombinierter Form als eine einzelne Lippe 14, zusätzlich Stützstrukturen aufweisen.

Der in Fig. 2 dargestellte Maskenwulst 1 weist in einem Seitenbereich 12 der Stützlippe 143 einen Abschnitt 16 auf, welcher dazu ausgebildet und eingerichtet ist, einen Schlauch 3, welcher zwischen Maskenwulst 1 und der Gesichtshaut 4 eines Anwenders angeordnet sein kann, zu führen. Ist kein Schlauch 3 zwischen Maskenwulst 1 und Gesichtshaut 4 des Anwenders angeordnet, so dichtet die Lippe 14 - bei Ausführungsformen mit zwei Lippen 14 primär die Dichtlippe 142 - den Maskenwulst 1 auf der Gesichtshaut 4 ab. Es kann durchaus vorkommen, dass - beispielsweise bei falscher Tragweise oder falsch gewählter Größe des Patienteninterface 2 beziehungsweise des Maskenwulstes 1 - auch die Stützlippe 143 zumindest stellenweise auf der Gesichtshaut 4 des Anwenders aufliegt und/oder diese berührt.

Der Maskenwulst 1 mit dem Abschnitt 16 ist also als Maskenwulst zur Anwendung zusammen mit einem Schlauch 3, beispielsweise für eine Magensonde oder ähnliches, gedacht. Darüber hinaus kann der Maskenwulst 1 aber auch ohne Schlauch 3 eingesetzt werden, ohne einen weniger bequemen Sitz oder eine geringere Abdichtung gegenüber Masken aufzuweisen, welche nur für eine Verwendung ohne Schlauch 3 konstruiert sind. Der gezeigte Maskenwulst 1 ist beispielhaft gezeigt als Maskenwulst für eine Gesichtsmaske für die nicht-invasive Beatmung, einige technische Merkmale können aber genauso auch auf andere Maskentypen, zum Beispiel für Nasalmasken oder andere Atemmasken.

Bei Positionieren eines Schlauches 3 zwischen Gesichtshaut 4 und der Dichtlippe 142 des Maskenwulstes 1, so dass der Schlauch 3 unter beziehungsweise entlang des Abschnittes 16 verläuft, legt sich der Abschnitt 16 auf und/oder um den Schlauch 3. Dabei wird die dazwischenliegende Dichtlippe 142 über den Schlauch 3 so verformt, dass die Dichtlippe 142 am Schlauch 3 verläuft, diesen zumindest stellenweise bedeckt und somit weitestgehend abdichtend ist. So kann, insbesondere gegenüber einem Maskenwulst ohne den für die Schlauchführung gedachten Abschnitt 16, eine Leckage bei Verwendung eines Schlauches 3 zwischen dem Maskenwulst 1 und der Gesichtshaut des Anwenders minimiert werden. In der beispielhaften Ausführungsform aus Fig. 2 ist der Abschnitt 16 als Ausschnitt 161 ausgeführt, im Ausschnitt 161 weist die Stützlippe 143 also kein Material auf. Somit ist die Oberfläche der Stützlippe 143 im Abschnitt 16 unterbrochen. Der Abschnitt 16 kann in manchen Ausführungsformen auch als Dünnstelle ausgeführt sein, sodass der Abschnitt 16 eine geringere Materialstärke als die umgebenden Bereiche der Lippe aufweist. Die Breite des Ausschnittes 161 ist vorzugsweise größer als der Durchmesser des Schlauches 3, sodass der Ausschnitt 161 den Schlauch 3 aufnehmen kann. Bei manchen Ausführungsformen wird auch ein Teil der Dichtlippe 142, welche zwischen dem Ausschnitt 161 und dem Schlauch 3 angeordnet ist, ebenfalls vom Ausschnitt 161 aufgenommen. Der Ausschnitt 161 verformt die Dichtlippe 142 so, dass diese sich zum Teil um den Schlauch 3 legt und an den Kontaktstellen mit dem Schlauch 3 sowie der Gesichtshaut 4 abdichtet. Die Dichtlippe 142 wird also entlang des Schlauches 3 geführt und kann abseits davon dichtend an der Gesichtshaut 4 des Anwenders anliegen.

Der Maskenwulst 1 kann ferner in manchen beispielhaften Ausführungsformen auch mehrere, optional in der Form und Ausführung verschiedene, Abschnitte 16 aufweisen. Beispielsweise kann jeder Seitenbereich 12 einer Lippe 14, beispielsweise der Stützlippe 143, zumindest einen Abschnitt 16 aufweisen. Zum Beispiel kann auch jeder Seitenbereich 12 der Stützlippe 143 auch zwei und mehr Abschnitte 16 aufweisen, beispielsweise mit unterschiedlichen Ausmaßen für verschiedene Schlauchdurchmesser. Durch Abschnitte 16 auf jeder der beiden Seitenbereiche 12 kann beispielsweise je nach Lage und Anforderung ein Schlauch 3 von links und/oder rechts unter dem Maskenwulst 1 geführt werden.

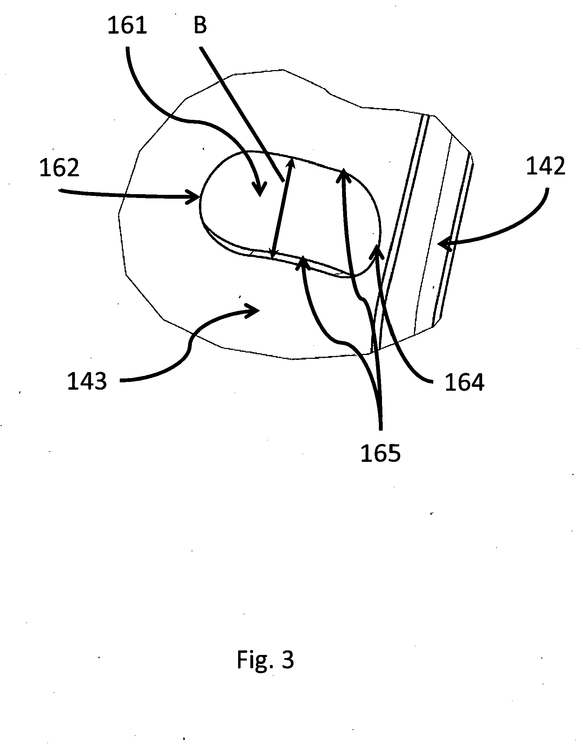

In Fig. 3 ist der als Ausschnitt 161 ausgeführte Abschnitt 16 des Maskenwulstes 1 ausschnittsweise vergrößert dargestellt. Der Abschnitt 16 erstreckt sich von einem Ende 162, welches in Richtung Übergangsbereich 21 angeordnet ist bis hin zu einem zweiten Ende 164, welches in Richtung der zentralen Öffnung 15 angeordnet ist. Begrenzt wird der Ausschnitt 161 zudem durch die Seiten 165. Der Ausschnitt 161 wird dadurch gekennzeichnet, dass der Ausschnitt 161 innerhalb der Begrenzung durch die Enden 162, 164 sowie der Seiten 165 kein Material aufweist, also eine Öffnung in der Stützlippe 143 darstellt. In der in Fig. 3 gezeigten, beispielhaften Ausführungsform des Ausschnittes 161 sind beide Enden 162 und 164 abgerundet und halbkreisförmig ausgeführt. Die Seiten 165 sind im Wesentlichen gerade und verlaufen parallel zu einander. In anderen beispielhaften Ausführungsformen können die Seiten 165 und/oder die Ende 162, 164 auch andere Formen annehmen. Beispielsweise können die Enden 162 und/oder 164 unabhängig voneinander auch abgeflacht sein oder sonstige Geometrien und Freiformen aufweisen. Die Seiten 165 können in manchen Ausführungsformen auch unabhängig voneinander einen kurvenförmigen Verlauf aufweisen, beispielsweise stellenweise in den Ausschnitt 161 hineinragen oder eine Ausbuchtung aufweisen. Je nach Länge des Ausschnitts 161, also der Ausdehnung von Ende 162 zu Ende 164, ist es möglich, dass die Seiten 165 nicht eindeutig erkennbar sind, sondern in der Form des Endes 162 und/oder 164 übergehen. Ist der Ausschnitt 161 beispielsweise als kreisrundes Loch ausgebildet so können die Enden 162 und 164 als Halbkreise angesehen werden, sodass keine Seiten 165 eindeutig erkennbar sind. Sind sowohl Ende 162 als auch Ende 164 geschlossen, der Ausschnitt 161 ist also nicht zur zentralen Öffnung 15 hin offen, so legt sich beispielsweise auch das Ende 164 über den Schlauch 3.

Die Seiten 165 weisen im Wesentlichen einen Abstand B voneinander auf. Dieser Abstand B ist im Bereich des Außendurchmessers des Schlauches 3, bevorzugt aber größer. In manchen Ausführungsformen ist der Abstand B auch kleiner als der Durchmesser des Schlauches 3. In manchen Ausführungsformen sind auf dem Maskenwulst 1 auch mehrere Abschnitte 16 beziehungsweise Ausschnitte 161 angeordnet, welche unterschiedliche Abstände B der Seiten 165 voneinander aufweisen, und somit ausgebildet sind Schläuche verschiedener Durchmesser aufzunehmen. Auch ist denkbar, dass der Abstand B in manchen Ausführungsformen zum Ende 162 hin beispielsweise kleiner wird. So kann zum Beispiel ein Ausschnitt 161 auch verschiedene Schlauchdurchmesser aufnehmen.

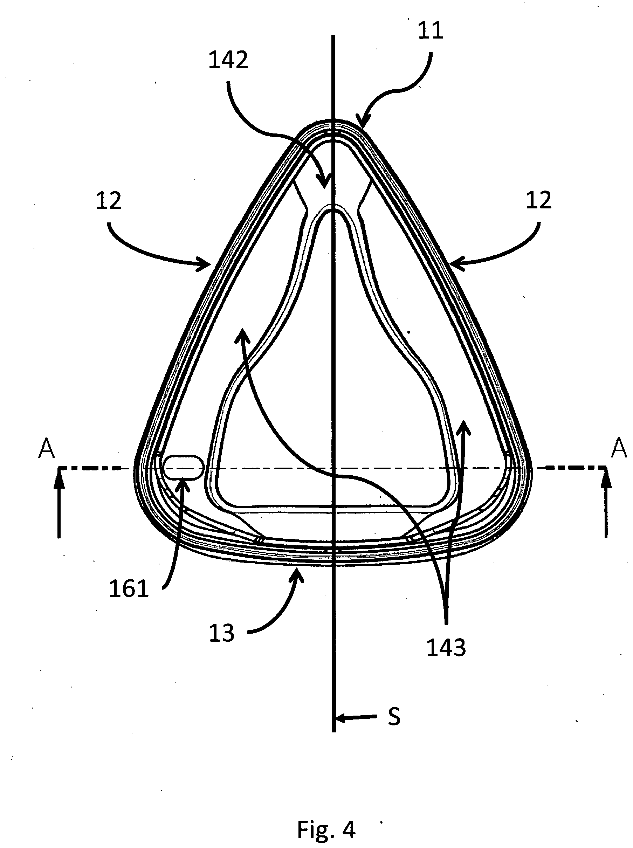

Fig. 4 zeigt den beispielhaften Maskenwulst in einer Rückansicht, also mit Blick auf den als Maskenkörperanschluss 211 ausgeführten Übergangsbereich 21. In der gezeigten beispielhaften Ausführungsform weist die Stützlippe 143 einen Ausschnitt 161 in dem Seitenbereich 12, nahe dem Basisbereich 13, auf. Beispielhaft gezeigt ist eine Ausführungsform, bei der die Seitenbereiche 12 der Stützlippe 143 im Nasenrückenbereich 11 sowie dem Basisbereich 13 nicht miteinander verbunden sind. In Fig. 4 ist weiter die Ebene S eingezeichnet, welche durch den Nasenrückenbereich 11 und den Basisbereich 13 verläuft. In einigen Ausführungsformen sind im Maskenwulst 1 zumindest zwei Abschnitte 16 angeordnet, welche sich gemäß der Ebene S gegenüberliegen und zum Beispiel die gleiche Geometrie und Ausmaße (Länge und Breite) aufweisen. Beispielsweise können auch zwei oder mehr Abschnitte 16, beispielsweise unterschiedlicher Geometrie und Ausmaße, in einem Seitenbereich 12 angeordnet sein, welche entsprechend an der Ebene S gespiegelt im gegenüberliegenden Seitenbereich 12 ebenfalls angeordnet sind.

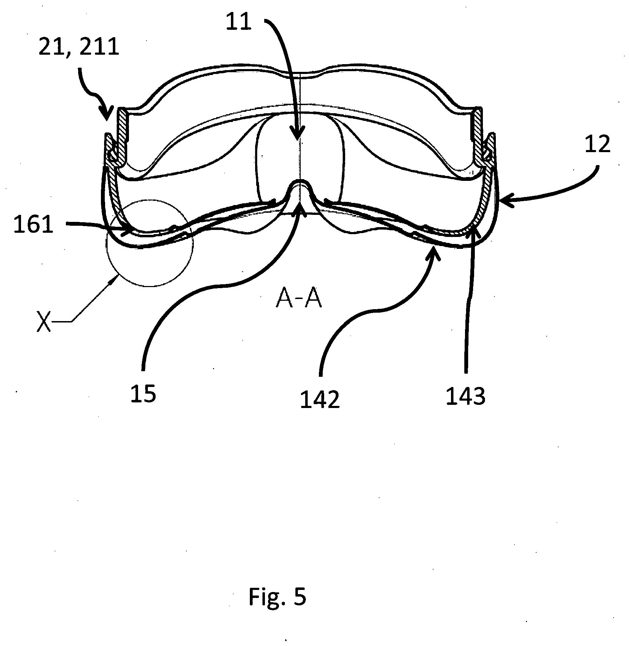

In Fig. 4 ist weiter ein Schnitt A-A unter anderem durch den Ausschnitt 161 eingezeichnet, welcher in Fig. 5 dargestellt ist. In Fig. 5 sieht man den in Teilen parallelen Verlauf der Dichtlippe 142 und der Stützlippe 143 vom Übergangsbereich 21 bzw. dem Maskenkörperanschluss 211 zur zentralen Öffnung 15. In der beispielhaften Ausführungsform verläuft die Dichtlippe 142 außen, sodass sie auf der Gesichtshaut 4 eines Anwenders aufliegen kann. Bei einer Ausführungsform mit zwei Lippen 14 ist die äußere Lippe 14 also die Lippe 14, welche von dem geometrischen Schwerpunkt des Maskenwulstes mit dem größten Flächenanteil am weitesten entfernt ist. Die Materialstärke der Dichtlippe ist im Wesentlichen konstant geringer als die Materialstärke der Stützlippe 143. Die Stützlippe 143 weist beispielhaft eine sich vom Übergangsbereich 21 in Richtung der zentralen Öffnung 15 graduell verringernde Materialstärke auf, so kann die Stützlippe 143 den Maskenwulst 1 abstützen. Die geringere Materialstärke zur zentralen Öffnung hingegen ermöglicht Beispielsweise eine Stützung der Dichtlippe 142 auf dem Gesicht des Anwenders ohne einen unbequemen Druck auszuüben. Die Materialstärke und/oder Steifigkeit bzw. Festigkeit der Stützlippe sollte dennoch groß genug sein, damit die Dichtlippe 142 über den Schlauch 3 geführt werden kann. In manchen Ausführungsformen dient die Stützlippe 143 zudem dazu - auch ohne Verwendung eines Schlauches 3 - die Dichtlippe 142 auf der Gesichtshaut 4 des Anwenders dicht auf liegen zu lassen und gegen Undichtigkeiten, beispielsweise bei hohen Beatmungsdrücken, zu stützen. In manchen Ausführungsformen ist der Maskenwulst 1 aus einem Material gefertigt, wobei die unterschiedlichen mechanischen Eigenschaften primär durch eine Variation der Geometrie und der Materialstärke erreicht werden. In manchen Ausführungsformen können Teile des Maskenwulstes 1 aber auch aus verschiedenen Materialien gefertigt sein. Beispielsweise kann für die Dichtlippe 142 ein elastischeres/dehnbareres Material als für die Stützlippe 143 gewählt werden. Die Stützlippe 143 kann im Gegenzug aus einem eher steifen Material bestehen. Dadurch kann beispielsweise eine bessere Stützwirkung und/oder Formstabilität der Stützlippe 143 erreicht werden. Auch kann durch eine sehr dehnbare Dichtlippe 142, die Dichtlippe 142 einfacher dichtanliegend um den Schlauch 3 geführt werden, wobei der Ausschnitt 161 der Stützlippe 143 dazu beiträgt, dass sich die Dichtlippe 142 zumindest teilweise um den Schlauch 3 legt.

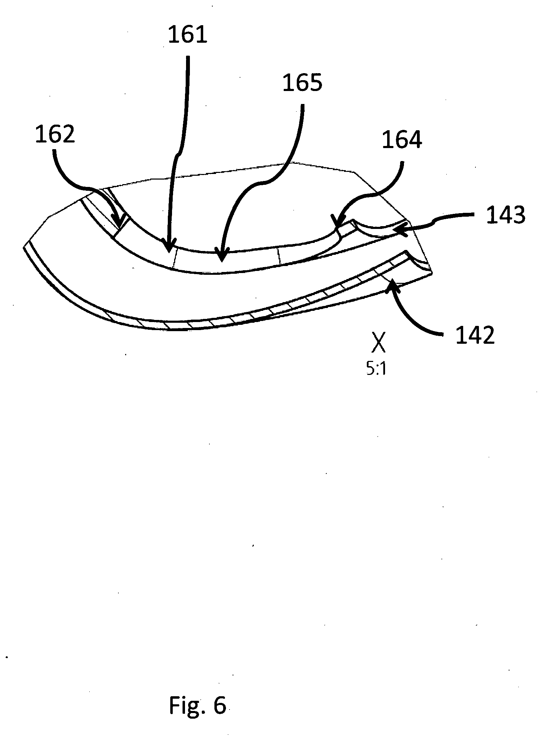

Fig. 6 zeigt den mit X markierten Teil der Fig. 5 im Detail. Die außenliegende Dichtlippe 142 weist eine geringere Materialstärke als die innenliegende Stützlippe 143 auf. Beispielhaft ist ein abrupter oder stufenförmiger Übergang von der Stützlippe 143 zum Ausschnitt 161 zu erkennen. In manchen Ausführungsformen kann dieser Übergang auch fließend ausgeführt sein, dass sich also die Materialstärke graduell in Richtung Mittelpunkt des Ausschnitts verringert und schließlich in den von Material freien Ausschnitt übergeht.

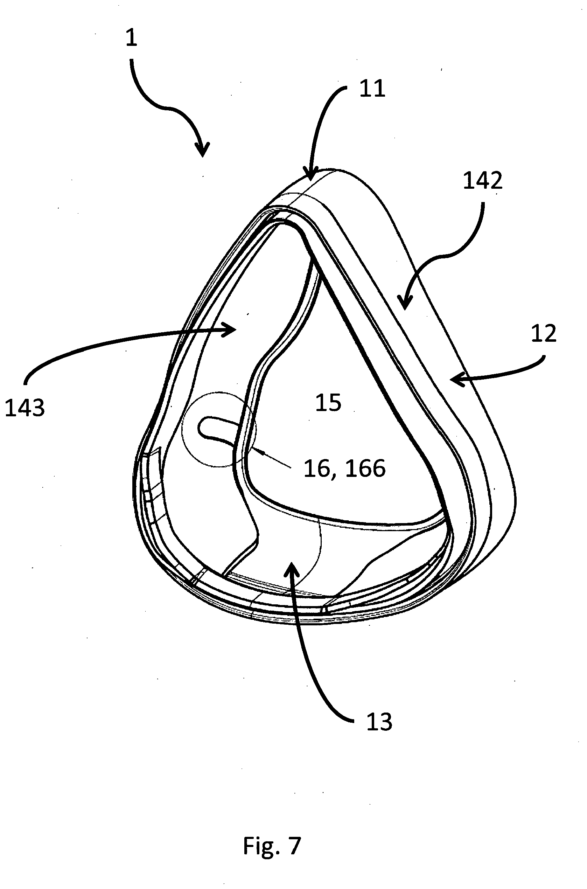

Die Fig. 7 bis 11 zeigen eine weitere.beispielhafte Ausführungsform des Maskenwulstes 1. Der Maskenwulst 1 entspricht dabei in großen Teilen dem zu Figuren 2 bis 6 beschriebenen Maskenwulst. Im Unterschied zu dem vorhergehend beschriebenen Maskenwulst ist der Abschnitt 16 hier als eine Dünnstelle 166 ausgeführt. Im Gegensatz zu dem Ausschnitt 161 stellt die Dünnstelle 166 keine Unterbrechung der Stützlippe 143 dar, sondern weist gegenüber der restlichen Stützlippe 143 eine deutlich geringere Materialstärke auf. In manchen Ausführungsformen ist die Dünnstelle 166 zudem aus einem anderen, beispielsweise elastischerem Material als die Stützlippe 143.

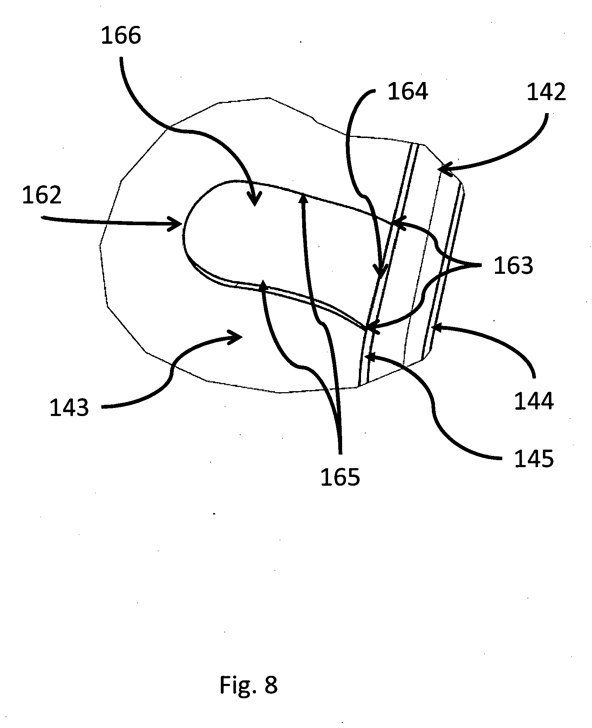

In der beispielhaft gezeigten Ausführungsform reicht das Ende 164 der Dünnstelle 166 bis an die Kante 145 der Stützlippe 143 zur zentralen Öffnung 15, wie in Fig. 8 beispielhaft eingezeichnet. Dadurch, dass das Ende 164 der Dünnstelle nicht abgerundet ist, sind auch die Endpunkte 163 des Dünnstelle 166 zu erkennen. Es ist zudem auch denkbar, dass das Ende 164 der Dünnstelle 166 nicht bis ganz an die Kante 145 reicht.

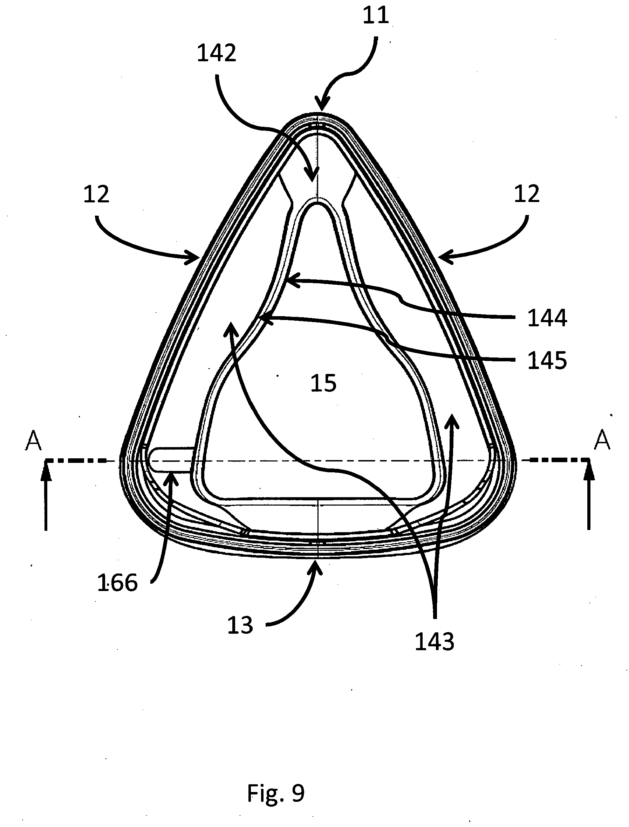

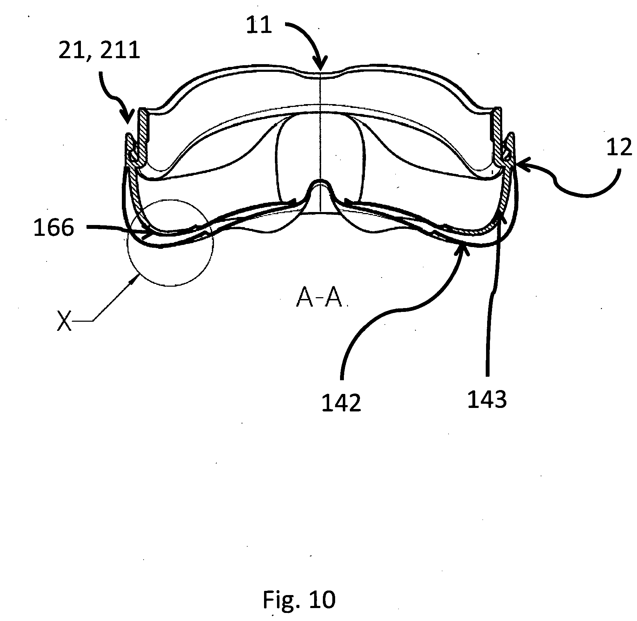

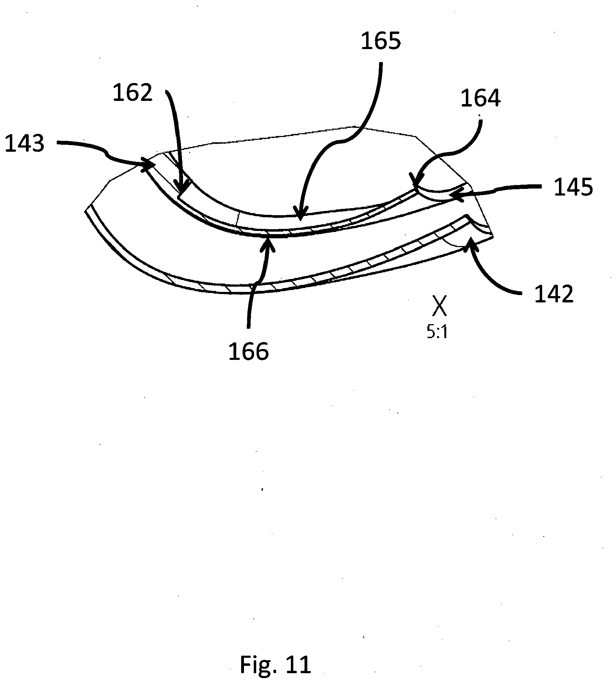

Die Fig. 11 zeigt das Detail X der Fig. 10 aus dem Schnitt A-A (Fig. 9) durch den Maskenwulst 1. Die Materialstärke der Stützlippe 143 ändert sich dort beispielhaft abrupt beim Übergang in die Dünnstelle 166. Der hier gezeigte abrupte Übergang verläuft stufenförmig auf der inneren Seite der Stützlippe 143, also bei Anwendung der vom Gesicht des Anwenders abgewandten Seite. Der Übergang kann aber auch stufenförmig auf der äußeren, also dem Gesicht des Anwenders zugewandten Seite verlaufen. Auch ein stufenförmiger Übergang auf beiden Seiten der Stützlippe 143 kann beispielsweise möglich sein.

In manchen Ausführungsformen kann der Übergang zur Dünnstelle 166 auch durch eine graduelle Verringerung der Materialstärke ausgebildet sein. Den Beginn der Dünnstelle 166 kann beispielsweise der Beginn oder das Ende der graduellen Verringerung darstellen. Auch eine Definition eines Übergangsbereiches zur Dünnstelle 166 ist möglich. Die Dünnstelle 166 ist in der Regel mit einer im Wesentlichen homogenen Materialstärke ausgeführt, in manchen Ausführungsformen kann die Materialstärke innerhalb der Dünnstelle 166 aber auch variieren.

Die Dünnstelle 166 weist gegenüber der restlichen Stützlippe 143 eine deutlich geringere Steifigkeit und/oder Festigkeit, z.B. zusammenhängend mit der geringeren Materialstärke oder auch durch Wahl eines anderen Materials, auf und ist im Vergleich zu der restlichen Stützlippe 143 dehnbarer. Diese geringere Steifigkeit und/oder höhere Dehnbarkeit ermöglicht es, dass die Stützlippe 143 sowohl die Dünnstelle 166 als auch die Dichtlippe 142 zumindest teilweise um den Schlauch 3 legen bzw. führen kann. Durch die steifer und/oder dicker ausgeführten Seitenbereiche an den Seiten 165 und dem Ende 162 der Stützlippe 143 wird die Dichtlippe 142 so verformt, dass sich ein Teil der Dichtlippe 142 auf den Schlauch 3 legt und an den Seiten des Schlauches 3 bis zur Gesichtshaut 4 des Anwenders gedrückt bzw. gezogen wird und dort anliegt.

Sind mehrere Abschnitte 16 auf dem Maskenwulst 1 angeordnet, so können diese zum Teil als Ausschnitte 161 und zum Teil auch aus Dünnstellen 166 ausgebildet sein. In manchen Ausführungsformen ist es möglich, dass beispielsweise unter beziehungsweise parallel verlaufend zu einem Ausschnitt 161 in der Stützlippe 143 eine zusätzliche Dünnstelle 166 in der Dichtlippe 142 angeordnet ist. In manchen Ausführungsformen kann die Dichtlippe 142 einen Ausschnitt 161 aufweisen und die Stützlippe 143 eine Dünnstelle 166. Weiter ist es zum Beispiel auch denkbar, dass Dichtlippe 142 und Stützlippe 143 übereinanderliegende Dünnstellen 166 und/oder Ausschnitte 161 aufweisen. Auch denkbar ist, dass die übereinanderliegenden Abschnitte unterschiedliche Geometrien und/oder Größen aufweisen. Beispielsweise kann der Abschnitt 16 der Dichtlippe 142 breiter und/oder länger (also Abstand zwischen den Enden 162 und 164) als der Abschnitt 16 der Stützlippe 143 ausgeführt sein. Auch kann es möglich sein, dass der Abschnitt 16 der Stützlippe 143 breiter und/oder länger als der Abschnitt 16 der Dichtlippe 142 ist.

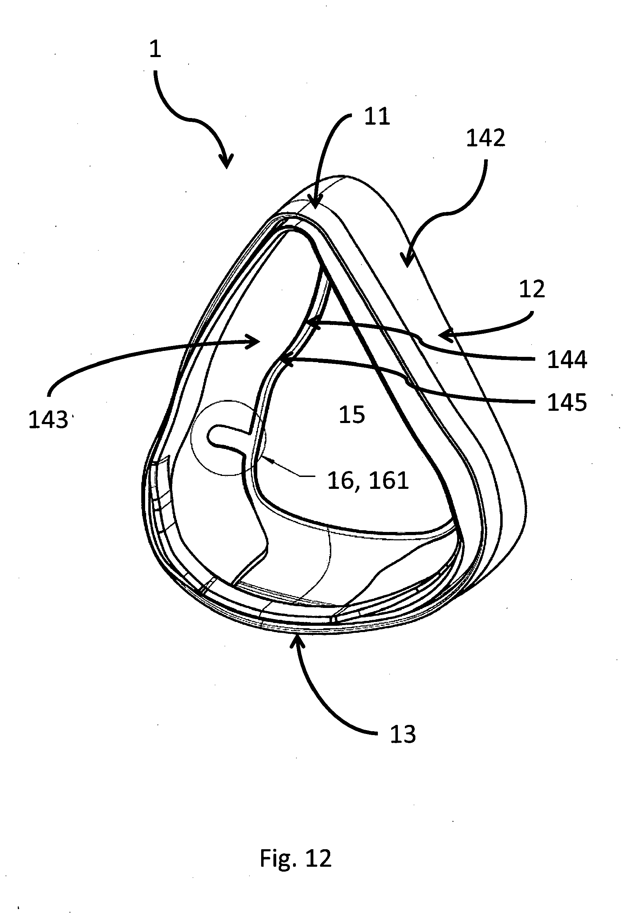

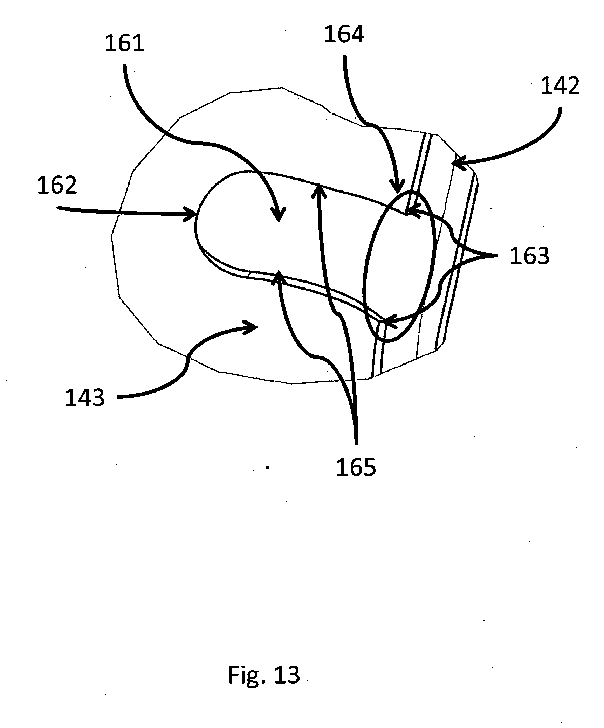

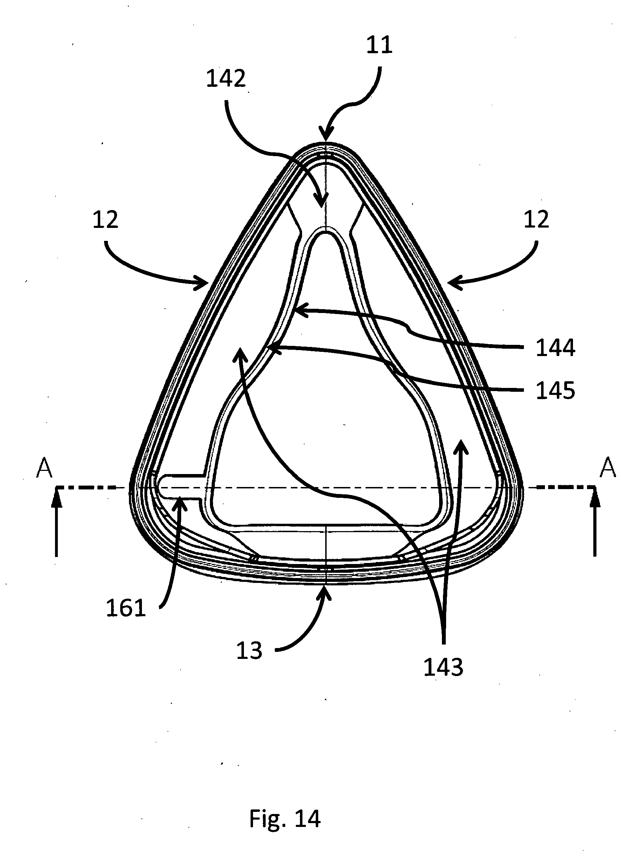

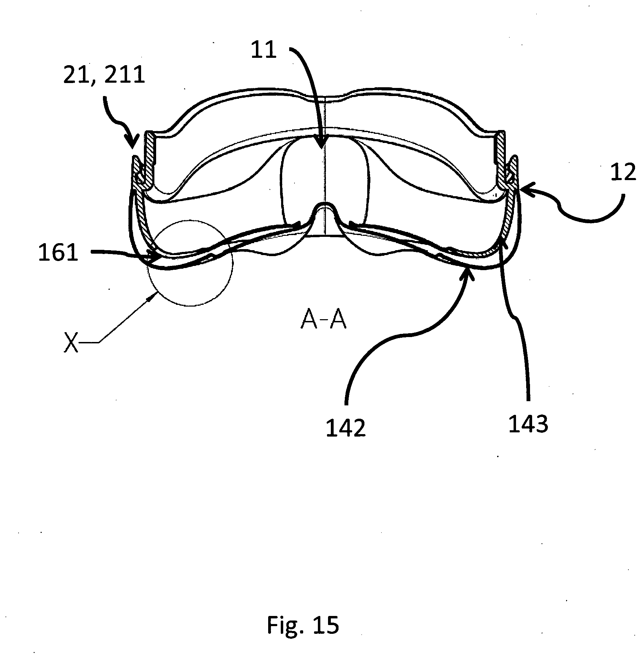

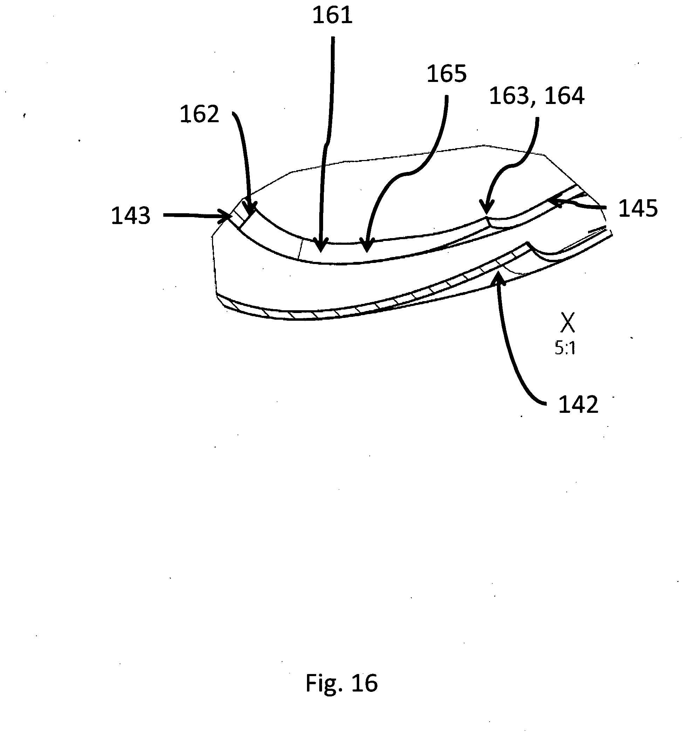

In den Figuren 12 bis 16 ist eine weitere beispielhafte Ausführungsform des Maskenwulstes 1 schematisch dargestellt. Zu den vorhergehend beschriebenen Ausführungsformen unterscheidet sich die in den Fig. 12 bis 16 gezeigte Ausführungsform dadurch, dass der Abschnitt 16 ein Ausschnitt 161 ist, welcher zur zentralen Öffnung 15 hin offen ist. Das Ende 164 des Ausschnittes 161 reicht also bis zur Kante 145 der Stützlippe 143, wobei die Endpunkte 163 des Ausschnittes 161 nicht direkt miteinander verbunden sind und sich zumindest dann nicht berühren, wenn keine mechanische Belastung - etwa durch Anlegen des Maskenwulstes 1 an das Gesicht eines Anwenders und festziehen der Bänderung - auf den Maskenwulst 1 wirkt.

Bei Verwendung eines Schlauches 3 zwischen Maskenwulst 1 und Gesichtshaut 4 des Anwenders, wird die Dichtlippe 142 durch den Ausschnitt 161 mindestens teilweise über den Schlauch 3 gestülpt beziehungsweise gedrückt. Die Dichtlippe 142 kann sich über den Schlauch 3 in den Abschnitt 16, hier als Ausschnitt 161, verformen und sich so zumindest teilweise um den Schlauch 3 legen.

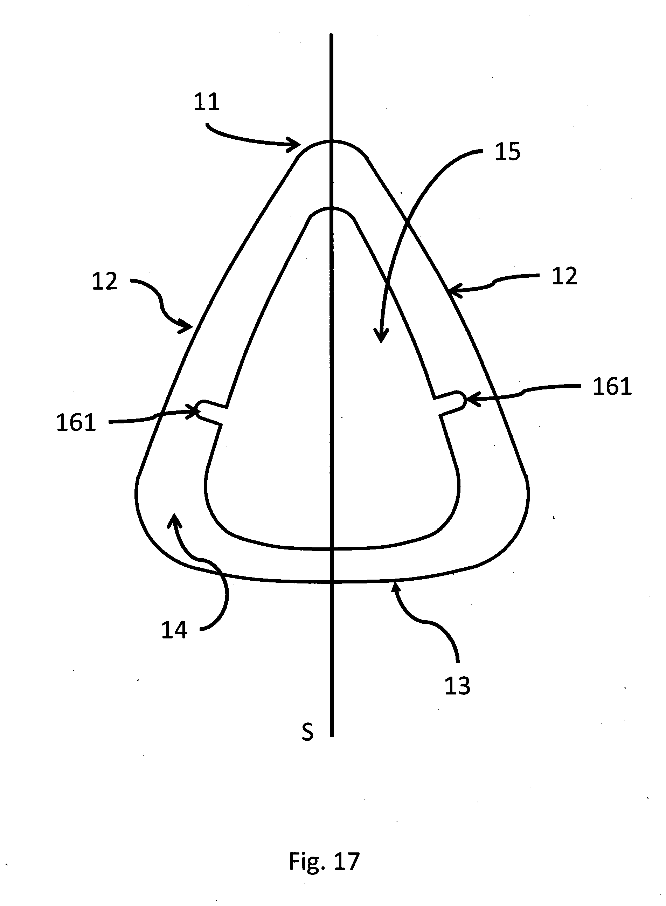

In Fig. 17 ist eine beispielhafte Ausführungsform des Maskenwulstes 1 in Frontansicht dargestellt. Der Maskenwulst 1 weist eine Lippe 14 auf, welche als kombinierte Dichtlippe 142 und Stützlippe 143 ausgeführt ist. In der gezeigten Ausfiihrungsform weist die Lippe 14 in den beiden Seitenbereichen 12 jeweils einen als Ausschnitt 161 ausgeführten Abschnitt 16 auf. Die beiden Ausschnitt 161 liegen sich gemäß der Ebene S durch den Basisbereich 13 und den Nasenrückenbereich 11 gegenüber. Die Abschnitte 161 sind zur zentralen Öffnung 15 hin offen und bilden so eine Unterbrechung der Oberfläche der Lippe 14. Bei Anwendung eines Schlauches 3 zusammen mit dem Maskenwulst 1 kann der Ausschnitt 161 den Schlauch 3 aufnehmen, so dass die Seiten 165 des Ausschnittes 161 an den Seiten des Schlauches entlang bis auf die Gesichtshaut 4 des Anwenders reichen. In manchen Ausführungsformen können die Endpunkte 163 des Ausschnittes 161 so geformt sein, dass diese auch ohne mechanische Belastung (auf den Maskenwulst) auf einander zulaufen, sich aber nicht berühren. Sind die Endpunkte 163 so geformt, dass diese beispielsweise einen Teilkreis bilden, so können diese sich unter den Schlauch 3 auf die Gesichtshaut 4 legen und dadurch die Leckage des Maskenwulstes 1 gegebenenfalls weiter verringern.

### Bezugszeichenliste

- 1: Maskenwulst
- 2: Patienteninterface
- 3: Schlauch
- 4: Gesichtshaut
- 11: Nasenrückenbereich
- 12: Seitenbereich
- 13: Basisbereich
- 14: Lippe
- 15: Öffnung
- 16: Abschnitt
- 21: Übergangsbereich
- 141: Oberfläche
- 142: Dichtlippe
- 143: Stützlippe
- 161: Ausschnitt
- 162: Ende
- 163: Endpunkt
- 164: Ende
- 165: Seite
- 166: Dünnstelle
- 211: Maskenkörperanschluss

## Patentansprüche

1. Maskenwulst (1) für ein Patienteninterface (2), mit zumindest einer Lippe (14), wobei die zumindest eine Lippe sich ausgehend von einem Übergangsbereich (21) zu einer zentralen Öffnung (15) hin erstreckt, wobei die mindestens eine Lippe (14) die zentrale Öffnung (15) umrandet, wobei mindestens eine Lippe (14) zumindest einen Abschnitt (16) aufweist, welcher zur Führung eines Schlauches (3) zwischen Maskenwulst (1) und der Gesichtshaut (4) eines Anwenders dient, **dadurch gekennzeichnet, dass** der Maskenwulst (1) zumindest zwei Lippen (14) aufweist, wobei mindestens eine Lippe (14) als Dichtlippe (142) und mindestens eine Lippe (14) als Stützlippe (143) ausgebildet ist, wobei die Stützlippe (143) den mindestens einen Abschnitt (16) aufweist.

2. Maskenwulst (1) nach zumindest einem der vorhergehenden Ansprüche, **dadurch gekennzeichnet, dass** der Übergangsbereich (21) ein Maskenkörperanschluss (211) ist.

3. Maskenwulst (1) nach zumindest einem der vorhergehenden Ansprüche, **dadurch gekennzeichnet, dass** das Material der Dichtlippe (142) im Bereich des Abschnittes (16) verformbar ist und sich mindestens teilweise dem Schlauch anpasst, wobei die Dichtlippe (142), ohne Verwendung eines Schlauches (3) zwischen Maskenwulst (1) und Gesicht des Patienten, dichtend auf dem Gesicht des Patienten aufliegt.

4. Maskenwulst (1) nach zumindest einem der vorhergehenden Ansprüche, **dadurch gekennzeichnet, dass** der Abschnitt (16) ein Ausschnitt (161) ist, welcher die Oberfläche (141) der Stützlippe (143) mindestens stellenweise unterbricht.

5. Maskenwulst (1) nach zumindest einem der vorhergehenden Ansprüche, **dadurch gekennzeichnet, dass** die Dichtlippe (142) außen angeordnet ist und eine weitestgehend geringere Materialstärke als die Stützlippe (143) aufweist, wobei die Stützlippe (143) innen angeordnet ist.

6. Maskenwulst (1) nach Anspruch 5, **dadurch gekennzeichnet, dass** der Abschnitt (16) zumindest ein Ende (164) in Richtung der zentralen Öffnung (15) sowie ein Ende (162) in Richtung zum Übergangsbereich (21) aufweist, wobei das Ende (162) des Abschnittes (16) abgerundet, bevorzugt halbkreisförmig, ist.

7. Maskenwulst (1) nach Anspruch 5, **dadurch gekennzeichnet, dass** der Ausschnitt (161) zur zentralen Öffnung (15) hin offen ist, wobei die Endpunkte (163) des zur Öffnung (15) gerichteten Endes (164) des Ausschnittes (161) voneinander beabstandet sind und sich die Seiten (165) des Ausschnitts (161) im Bereich des Endes (162) nicht berühren.

8. Maskenwulst (1) nach zumindest einen der vorhergehenden Ansprüche, **dadurch gekennzeichnet, dass** der Abschnitt (16) ausgebildet ist, über einem Schlauch (3) zu liegen und sich zumindest abschnittsweise der Schlauchform anzupassen.

9. Maskenwulst (1) nach Anspruch 5, **dadurch gekennzeichnet, dass** die Stützlippe (143) einen Nasenrückenbereich (11), zwei Seitenbereiche (12) sowie einen Basisbereich (13) aufweist, wobei sich die Stützlippe (143) von den Seitenbereichen (12) in Richtung zentrale Öffnung (15) erstreckt und im Bereich des Nasenrückenbereichs (11) und des Basisbereichs (13) nicht miteinander verbunden ist.

10. Maskenwulst (1) nach Anspruch 9, **dadurch gekennzeichnet, dass** die Stützlippe (143) mindestens zwei Abschnitte (16) aufweist, wobei je mindestens einer der mindestens zwei Abschnitte (16) sich von den Seitenbereichen (12) in Richtung der zentralen Öffnung (15) erstreckt.

11. Maskenwulst (1) nach Anspruch 9, **dadurch gekennzeichnet, dass** die Stützlippe (143) mindestens zwei Abschnitte (16) aufweist, welche sich, bezogen auf eine Ebene (S) durch den Nasenrückenbereich (11) und den Basisbereich (13), gegenüberliegen.

## Claims

1. A mask bead (1) for a patient interface (2), wherein the mask bead (1) comprises at least one lip (14), the at least one lip extending from a transition region (21) toward a central opening (15) and bordering the central opening (15), wherein at least one lip (14) comprises at least one portion (16) which is capable of guiding a tube (3) between the mask bead (1) and the facial skin (4) of a user, **characterized in that** the mask bead (1) comprises at least two lips (14), at least one lip (14) being configured as sealing lip (142) and at least one lip (14) being configured as supporting lip (143), wherein the supporting lip (143) comprises the at least one portion (16).

2. The mask bead (1) of at least one of the previous claims, **characterized in that** the transition region (21) is a mask body connector (211).

3. The mask bead (1) of at least one of the previous claims, **characterized in that** the material of the sealing lip (142) in a region of the portion (16) is deformable and at least partially adapts to the tube, wherein the sealing lip (142), without use of a tube (3) between the mask bead (1) and a face of the patient, bears sealingly on the face of the patient.

4. The mask bead (1) of at least one of the previous claims, **characterized in that** the portion (16) is a cutout (161), which at least in some places interrupts the surface (141) of the supporting lip (143).

5. The mask bead (1) of at least one of the previous claims, **characterized in that** the sealing lip (142) lies on an outside and has a substantially smaller material thickness than the supporting lip (143), wherein the supporting lip (143) lies on an inside.

6. The mask bead (1) of claim 5, **characterized in that** the portion (16) comprises at least one end (164) in a direction of the central opening (15) and one end (162) in a direction of the transition region (21), the end (162) of the portion (16) being rounded, preferably semicircular in shape.

7. The mask bead (1) of claim 5, **characterized in that** the cutout (161) is open toward the central opening (15), wherein the end points (163) of the end (164) of the cutout (161) which is directed toward the opening (15) are spaced apart from each other and wherein sides (165) of the cutout (161) do not touch in a region of the end (162).

8. The mask bead (1) of at least one of the previous claims, **characterized in that** the portion (16) is configured to lie over a tube (3) and to adapt to the tube shape at least in some regions.

9. The mask bead (1) of claim 5, **characterized in that** the supporting lip (143) comprises a nose bridge region (11), two side regions (12) and a base region (13) and extends from the side regions (12) in a direction of the central opening (15) and, in a region of the nose bridge region (11) and of the base region (13), is not connected to itself.

10. The mask bead (1) of claim 9, **characterized in that** the supporting lip (143) comprises at least two portions (16), at least one of the at least two portions (16) extending from the side regions (12) in a direction of the central opening (15).

11. The mask bead (1) of claim 9, **characterized in that** the supporting lip (143) comprises at least two portions (16) which are opposite each other in relation to a plane (S) through the nose bridge region (11) and the base region (13).

## Revendications

1. Jupe de masque (1) pour une interface de patient (2) avec au moins une lèvre (14), l'au moins une lèvre s'étendant vers une ouverture centrale (15) à partir d'une zone de transition (21), l'au moins une lèvre (14) entourant l'ouverture centrale (15), l'au moins une lèvre (14) présentant au moins une section (16), laquelle sert au passage d'un tuyau (3) entre la jupe de masque (1) et la peau du visage (4) d'un utilisateur, **caractérisée en ce que** la jupe de masque (1) présente au moins deux lèvres (14), au moins une lèvre (14) étant conçue comme lèvre d'étanchéité (142) et au moins une lèvre (14) étant conçue comme lèvre de support (143),
la lèvre de support (143) présentant l'au moins une section (16).

2. Jupe de masque (1) selon au moins l'une des revendications précédentes, **caractérisée en ce que** la zone de transition (21) est un raccordement de corps de masque (211).

3. Jupe de masque (1) selon au moins l'une des revendications précédentes, **caractérisée en ce que** le matériau de la lèvre d'étanchéité (142) est déformable dans la zone de la section (16) et s'ajuste au moins partiellement au tuyau, la lèvre d'étanchéité (142) reposant de façon étanche sur le visage du patient sans utilisation d'un tuyau (3) entre la jupe de masque (1) et le visage du patient.

4. Jupe de masque (1) selon au moins l'une des revendications précédentes, **caractérisée en ce que** la section (16) est une section (161) qui interrompt la surface (141) de la lèvre de support (143) au moins par endroits.

5. Jupe de masque (1) selon au moins l'une des revendications précédentes, **caractérisée en ce que** la lèvre d'étanchéité (142) est disposée à l'extérieur et présente une épaisseur de matériau largement inférieure à celle de la lèvre de support (143), la lèvre de support (143) étant disposée à l'intérieur.

6. Jupe de masque (1) selon la revendication 5, **caractérisée en ce que** la section (16) présente au moins une extrémité (164) dans la direction de l'ouverture centrale (15) ainsi qu'une extrémité (162) dans la direction de la zone de transition (21), l'extrémité (162) de la section (16) étant arrondie, de préférence semi-circulaire.

7. Jupe de masque (1) selon la revendication 5, **caractérisée en ce que** la section (161) est ouverte vers l'ouverture centrale (15), les points d'extrémité (163) de l'extrémité (164) de la section (161) orientée vers l'ouverture (15) étant à distance les uns des autres et les côtés (165) de la section (161) ne se touchant pas dans la zone de l'extrémité (162).

8. Jupe de masque (1) selon au moins l'une des revendications précédentes, **caractérisée en ce que** la section (16) est conçue pour être disposée au-dessus d'un tuyau (3) et pour s'ajuster au moins par section à la forme du tuyau.

9. Jupe de masque (1) selon la revendication 5, **caractérisée en ce que** la lèvre de support (143) présente une zone d'arête nasale (11), deux zones latérales (12) ainsi qu'une zone de base (13), la lèvre de support (143) s'étendant des zones latérales (12) en direction de l'ouverture centrale (15) et n'étant pas reliée dans la région de la zone d'arête nasale (11) et de la zone de base (13).

10. Jupe de masque (1) selon la revendication 9, **caractérisée en ce que** la lèvre de support (143) présente au moins deux sections (16), respectivement au moins l'une des au moins deux sections (16) s'étendant des zones latérales (12) en direction de l'ouverture centrale (15).

11. Jupe de masque (1) selon la revendication 9, **caractérisée en ce que** la lèvre de support (143) présente au moins deux sections (16), lesquelles sont en face l'une de l'autre relativement à un plan (S) par la zone d'arête nasale (11) et la zone de base (13).
